# EUROPEAN PATENT APPLICATION

(11) **EP 3 453 349 A1**
(43) Date of publication of application: **13.03.2019**
(21) Application number: 17020417.6
(22) Date of filing: 10.09.2017
(51) Int. Cl.: A61B 17/80, A61B 17/68

(54) **ORTHOPAEDIC IMPLANT**

(71) Applicant: Gotfried, Yechiel, 2636416 Kiryat Motzkin (IL)
(72) Inventor: Gotfried, Yechiel, 2636416 Kiryat Motzkin (IL)
(74) Representative: Dennemeyer & Associates S.A.

(57) **Abstract**

An apparatus for placement at an anatomical site, the apparatus including an orthopedic implant, which includes a front end, a rear end, a top, a bottom, a right side, a left side, and a central part connecting the right and left sides. The top surface of the implant has a top surface defining a first sloping surface running downwardly from the front end to the rear end of the implant, and the implant having a second sloping surface running downwardly from one side to the opposite side of the implant, and the first and second sloping surfaces are perpendicular to each other. At least a portion of the implant is shaped to define a porous matrix, the porous matrix being shaped to define a plurality of pores that extend through the porous matrix, the pores being sized to receive inserted bone graft material into the pores. At least the central part has a plurality of said pores therein, and the central part defines at least one fixation passageway that passes through the implant from the top of the implant to the bottom of the implant, the at least one fixation passageway having a diameter larger than an average diameter of the pores and is extending almost perpendicular to said second sloping surface running downwardly from one side to the opposite side of the implant, and is adapted to accept a fixation element.

## Description

### FIELD OF THE INVENTION

Applications of the present invention relate in general to orthopedic implants, and specifically, to orthopedic implant for changing, indirectly, an angular orientation and anatomical location of a bone.

### BACKGROUND

In some pathologies, the patella is too close to the trochlea of the femur, and/or is improperly aligned with respect to the trochlea. Multiple other pathologies relate to inappropriately aligned bones.

### SUMMARY OF THE INVENTION

Applications of the present invention include orthopadic implants for operating on a subject, in order to treat pathological conditions. For example, an orthopedic implant having a porous matrix may be inserted into an osteotomy in a tibia of the subject, bone graft material being coupled to the implant. By implanting the implant in the osteotomy, the patella of the subject is indirectly rotated about the axis of insertion, and a distance of the patella from the anterior distal portion of the femur (i.e., the trochlea of the femur) is indirectly increased. The orthopedic implant includes a front end, a rear end, a top, a bottom, a right side, and a left side. To facilitate the rotation of the patella, the distance between the top and the bottom is greater at one of the sides than at the other one of the sides. In addition to the porous matrix, the implant is typically shaped to define one or more passageways passing through the implant, the passageways facilitating the coupling of bone graft material to the implant.

There is therefore provided, in accordance with a first application of the present invention, apparatus for placement at an anatomical site, the apparatus including:
an orthopedic implant, including:
   a front end,
   a rear end,
   a top,
   a bottom,
   a right side,
   a left side, and
   a central part connecting the right and left sides,
a distance between the top and the bottom being (a) greater at a first location at one of the sides than at a second location opposite the first location at the other one of the sides, and (b) greater at a third location at the front end than at a fourth location opposite the third location at the rear end,
at least a portion of the implant being shaped to define a porous matrix, the porous matrix being shaped to define pores that extend through the porous matrix in at least each of: (a) a front-to-rear direction, (b) a left-to-right direction, and (c) a top-to-bottom direction.

For some applications the pores are multidirectional oriented.

For some applications the diameter of the pores of the porous matrix is about 0.5 mm.

For some applications the pores are adapted to receive inserted bone graft material inside the pores.

For some applications, at least one of the sides of the implant is shaped to define at least one passageway that passes through the side, the passageway having a diameter larger than an average diameter of the pores.

For some applications, the implant defines at least one fixation passageway that passes through the implant from the top of the implant to the bottom of the implant, the passageway having a diameter larger than an average diameter of the pores adapted to receive fixation means.

For some applications, the central part of the implant defines the porous matrix.

For some applications, the implant is shaped to define an H-shape, when viewed in a direction from the top to the bottom.

For some applications, at least 90 percent of a volume of the implant that includes the pores includes the porous matrix.

For some applications, at least a portion of the front end, the rear end, the top, the bottom, the right side, or the left side of the implant defines a portion of the porous matrix.

For some applications, the implant is for use with a tool and the implant further includes a connection interface that facilitates a connection of the implant to the tool.

For some applications, the connection interface is front-facing.

For some applications, the connection interface is shaped to define a threaded hole.

For some applications, the tool includes a rod that is fixed to the implant at the connection interface.

For some applications, a connection between the rod and the implant is configured to be breakable at the connection interface due to a size of the connection interface.

For some applications, the distance between the top and the bottom is at least 0.5 mm greater at the first location at one of the sides than at the second location opposite the first location at the other one of the sides, and the distance between the top and the bottom is at least 0.5 mm greater at the third location at the front end than at the fourth location opposite the third location at the rear end.

For some applications, the distance between the top and the bottom is greater at the front end than at respective, corresponding opposite locations on the rear end, on both of the left and the right sides of the implant.

For some applications, the apparatus further includes a bone graft material.

For some applications, the implant consists of a metal.

For some applications, the implant consists of a plastic.

For some applications, the implant consists of bone graft material.

There is further provided, in accordance with a second application of the present invention, apparatus for placement at an anatomical site, the apparatus including:
an orthopedic implant, including:
   a front end,
   a rear end,
   a top,
   a bottom,
   a right side,
   a left side, and
   a connection interface that facilitates a connection of the implant to the tool,
a distance between the top and the bottom being (a) greater at a first location at one of the sides at the front end than at a second location opposite the first location at the front end of the other one of the sides, and (b) greater at a third location at the front end than at a fourth location opposite the third location at the rear end, and
at least a portion of the implant being shaped to define a porous matrix, the porous matrix being shaped to define pores that extend through the porous matrix in at least each of: (a) a front-to-rear direction, (b) a left-to-right direction, and (c) a top-to-bottom direction.

It is noted that any of the various applications described hereinabove in the context of the first application of the present invention may be applied with the second application of the present invention as well.

### BRIEF DESCRIPTION OF THE DRAWINGS

Figs. 1A, 1B, 2A, 2B, 3, 4, 5, 6, 7 and 8 are schematic illustrations of an orthopedic implant for implanting in an anatomical site, Figs. 2A and 2B showing a cross section of the implant, in accordance with some applications of the present invention;
Fig. 9 is a schematic illustration of an orthopedic implant and a tool, in accordance with some applications of the present invention;
Fig. 10 is a schematic illustration of an orthopedic implant, in accordance with some applications of the present invention;
Fig. 11 is a schematic illustration of a portion of human anatomy;
Figs. 12-13 are schematic illustrations of an implant implanted in an osteotomy in a tibia of a subject, in accordance with some applications of the present invention; and

### DETAILED DESCRIPTION OF THE DRAWINGS

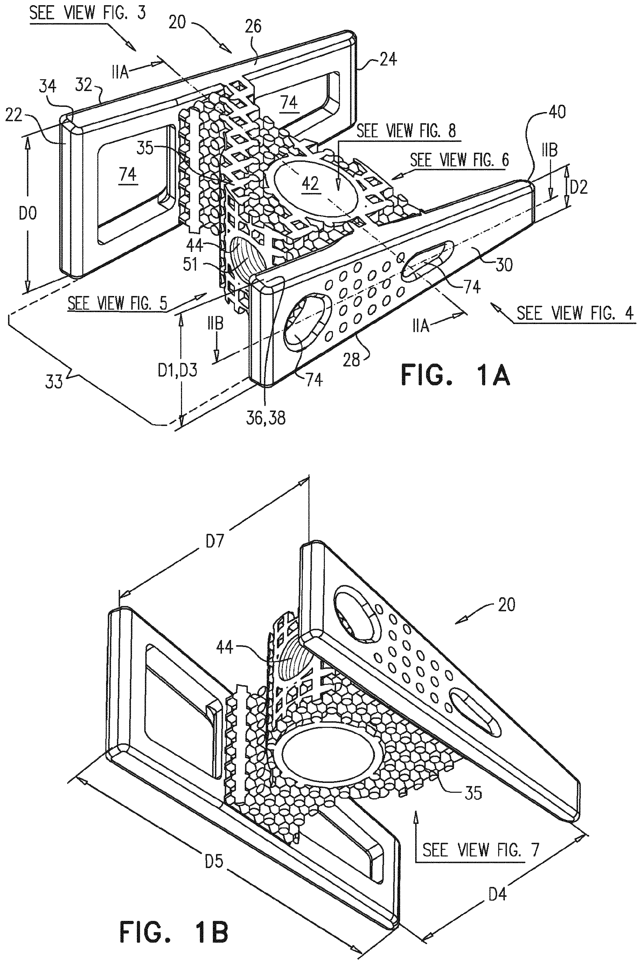

Reference is made to Figs. 1A-10, which are schematic illustrations of an orthopedic implant 20 for implanting in an anatomical site, in accordance with some applications of the present invention. Typically, the anatomical site is surrounded at least in part by one or more bones. For example, implant 20 may be implanted in a bone (e.g., an osteotomy, such as an osteotomy in a tibia), or a space between bones.

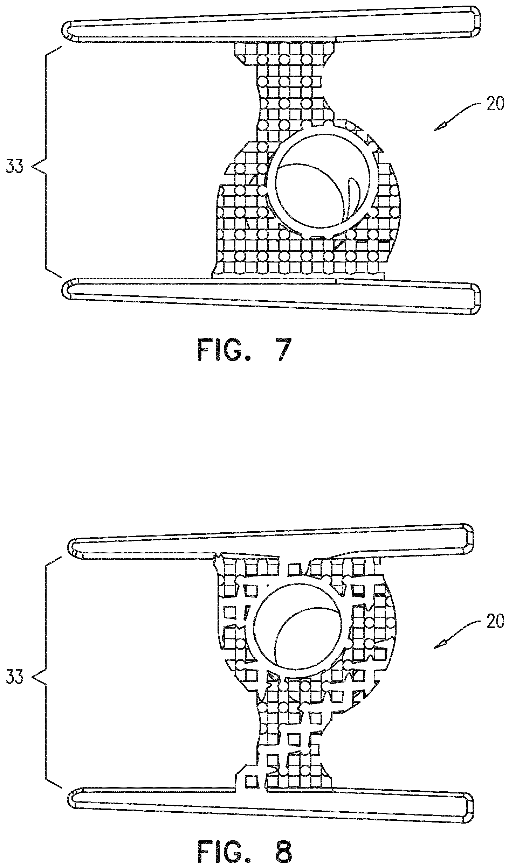

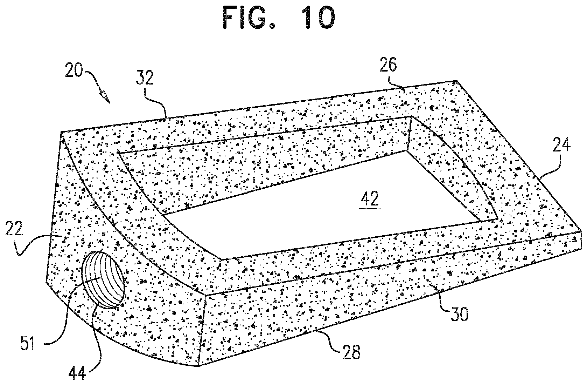

Implant 20 comprises a front end 22, a rear end 24, a top 26, a bottom 28, a right side 30, and a left side 32. Implant 20 typically additionally comprises a central part 33 connecting right and left sides 30 and 32 (as shown in Figs. 1-9, but not shown in Fig. 10). Thus, implant 20 of Figs. 1-9 is shaped to define an H-shape, when viewed in a direction from the top surface to the bottom surface (Fig. 8) .

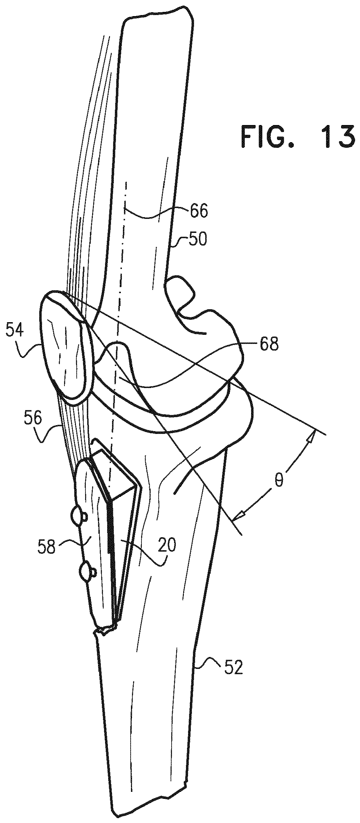

As further described hereinbelow, e.g., with reference to Fig. 13, one function of implant 20 is to change an angular orientation of one bone portion (i.e., part or all of a bone) with respect to another bone portion. To facilitate this function, a distance D0 between top 26 and bottom 28 at one of the sides is greater than a distance D1 between the top and bottom at the other side, along at least a portion of the sides. (For convenience, the distance between the top and bottom of the implant may be referred to below as the "height" of the implant.) In particular, D0 at a first location 34 at one of the sides is greater than D1 at a second location 36 opposite the first location at the other one of the sides. In some applications, D0 is greater than D1 along at least 80% of one of the sides. In some applications, for at least one instance of the first location and second location, D0 is at least 0.5 mm greater, e.g., at least 2.5 mm greater, and/or less than 20 mm greater, than D1. In other words, at at least one pair of opposite locations, the height at one of the sides is at least 0.5 mm greater and/or less than 20 mm greater than the height of the other one of the sides. Alternatively or additionally, at at least one pair of opposite locations, D0 is at least 0.5% greater than D1.

Typically, implant 20 is also shaped such that a distance D3 between the top and bottom at the front end is greater (e.g., 0.5% greater) than a distance D2 between the top and bottom at the rear end, along at least a portion of the ends. In particular, D3 at a third location 38 at the front end is greater than D2 at a fourth location 40 opposite the third location at the rear end. In some applications, D3 is greater than D2 along at least 80% of one of the ends. In general, this shape facilitates changing a second angular orientation of a bone portion, and/or facilitates the wedging of the implant within an anatomical site. In some applications, a distance D7 between the sides at the front end is greater than the distance D4 between the sides at the rear end.

Typically, bone graft material is inserted into the anatomical site along with implant 20, the pores are adapted to receive inserted bone graft material inside the pores. The bone graft material helping to stimulate bone formation in the anatomical site. (It is noted that in the context of the claims and specification of the present application, "bone graft material" includes any type of bone graft or bone graft substitute.) For some applications, at least a portion of implant 20, e.g., some or all of central part 33, is shaped to define a porous matrix, the porous matrix being shaped to define pores that extend through the porous matrix in at least each of: (a) a front-to-rear direction, (b) a left-to-right direction, and (c) a top-to-bottom direction. The pores may thus extend in many "diagonal" directions, i.e., combinations of (a), (b), and (c), which are not solely (a), (b), or (c). While not wishing to be limited by any particular dimensions for the pores, the inventor notes that the pores may be, for some applications, tens or hundreds of microns in size (e.g., in diameter). For some purposes, e.g., depending on the type of bone graft to be used, larger or smaller pores are suitable. The pores may have a 0.5 mm diameter.

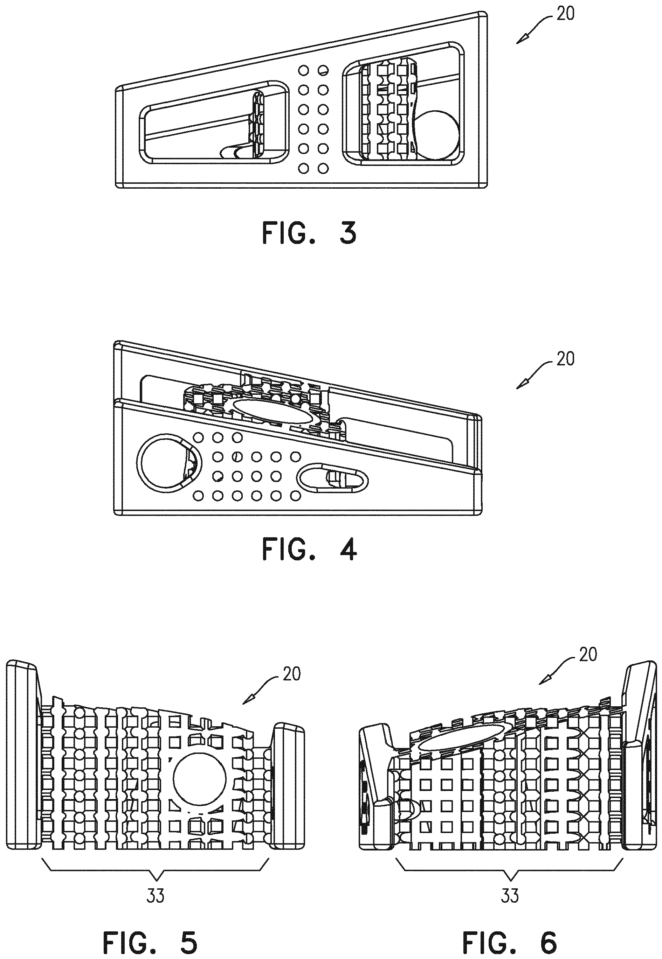

At least a portion of the front end, the rear end, the top, the bottom, the right side, or the left side of implant 20 typically defines a portion of the porous matrix. For example, Figs. 1A and 5 respectively show a portion of the top and bottom of the implant defining a portion of the porous matrix. Fig. 10 additionally shows the front end, the rear end, the right side, and the left side defining a portion of the porous matrix.

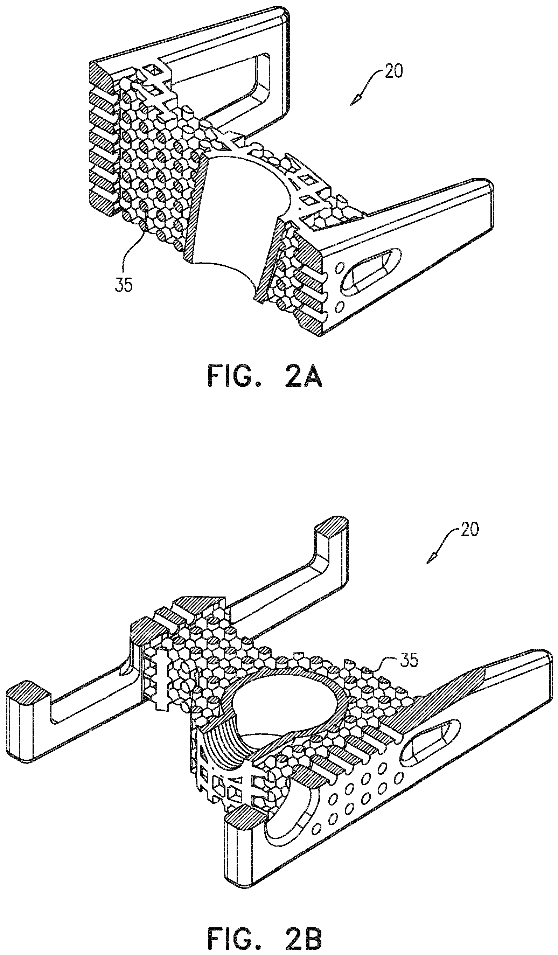

Typically, but not necessarily, implant 20 is additionally shaped to define one or more passageways 74 passing through the implant, and the bone graft material is disposed within the pores of the porous matrix and/or within passageways 74. Typically, at least one of the passageways 42 passes from the top to the bottom of the implant. Fixation passageway 42 typically have a diameter larger than an average diameter of the pores, adapted to accept fixation means as shown for example in Figs. 1A-B and 2A-B.

In some applications, at least 50%, for example, at least 80% (e.g., at least 90%) of the front end, rear end, top, bottom, right side, and/or left side is shaped to define openings to at least some of the passageways. Fig. 3 shows an example of this when implant 20 is viewed from the side. Similarly, Fig. 10 shows the implant having porous top, bottom, and side surfaces, and a large fixation central passageway 42, accepting fixation means like a screw in Fig. 13. Alternatively or additionally, at least 60 percent (e.g., 90 percent) of a volume of the implant that includes the pores comprises the porous matrix. The characteristics described in this paragraph typically allow for a relatively large amount of bone graft material to be inserted into the anatomical site. Alternatively or additionally, to facilitate insertion of bone graft material, the implant may be shaped to define a relatively large number of passageways, e.g., at least 5, 10, or more. Alternatively or additionally, the openings to the passageways may be relatively large, to facilitate insertion of bone graft material, and/or to facilitate access and/or coupling of a tool (e.g., an implantation tool) to the implant. For example, Figs. 1A-9 shows large lateral openings 74.

In some applications, some of the openings may be used for passing a drill-alignment tool therethrough. For example, alignment techniques described in US 2005/0010223 to Gotfried, which is incorporated herein by reference, and shown in Figs. 6 and 8 thereof, may be used for this purpose.

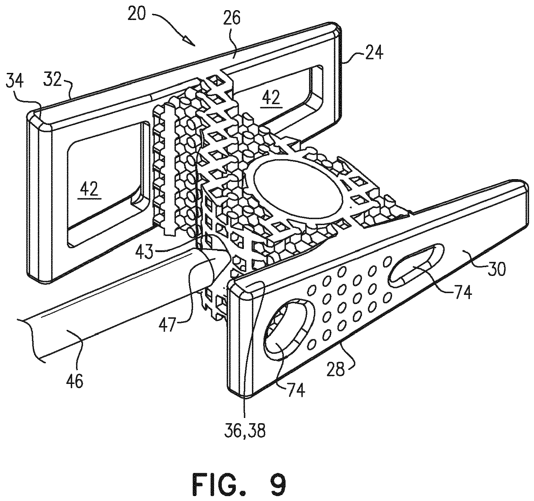

Typically, implant 20 comprises a connection interface 44, such as a threaded hole 51, which facilitates a connection of the implant to a tool, e.g., an implantation tool. (Although connection interface 44 is typically front-facing, it may also face in a different direction; for example, side-facing lateral openings 74 may be considered connection interfaces.) For some applications, e.g., as shown in Fig. 9, the tool comprises a rod 46 that is fixed to the implant at connection interface 44. A connection 43 between rod 46 and implant 20 is configured to be breakable at connection interface 44 due to a size of the connection interface. For example, a distal portion 47 of rod 46 may be narrowed, such that the physician holding the rod may be able to break the rod from the implant at connection 44 by applying up and down bending motion to the rod or by applying side to side bending motion to the rod.

In some applications, the implant consists entirely of a metal, e.g., a metallic alloy and/or titanium. In other applications, the implant consists entirely of a plastic (e.g., polyether ether ketone), or entirely of bone graft material.

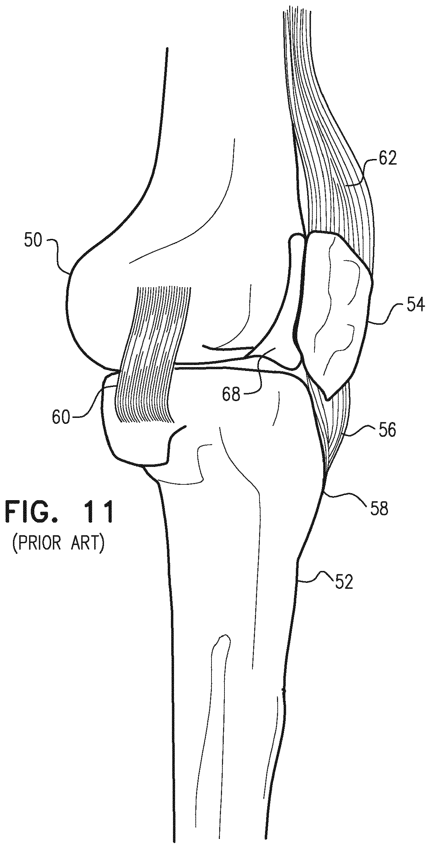

Reference is now made to Fig. 11, which is a schematic illustration of a portion of human anatomy. Fig. 11 shows the tibia 52, which includes the tibial tuberosity 58, which is a large oblong elevation on the proximal, anterior aspect of tibia 52. The tibia is joined to the femur 50 via, *inter alia,* the lateral collateral ligament 60. The patellar tendon 56 joins tibial tuberosity 58 to the patella 54, which, in turn, is joined to femur 50 via the quadriceps tendon 62. In some pathologies, the patella is too close to the trochlea 68 of the femur (i.e., the anterior distal portion of the femur), and/or is improperly aligned with respect to trochlea 68. Applications of the present invention address this pathology, as will now be described.

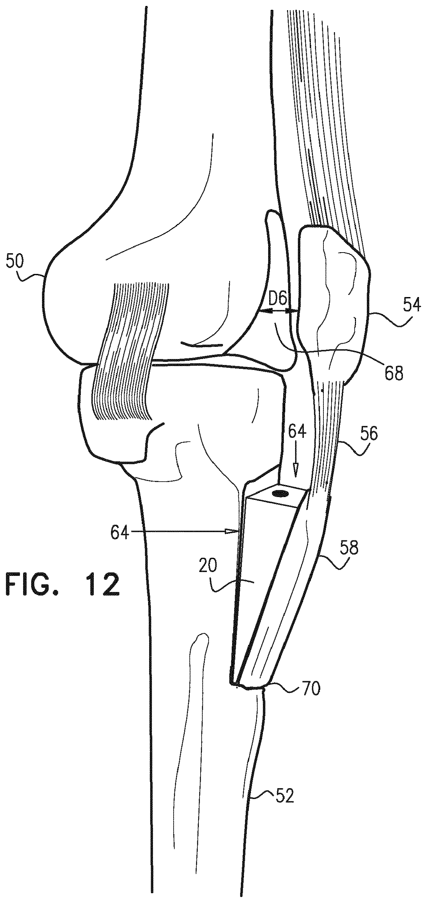

Reference is now made to Figs. 12-13 which are schematic illustrations of implant 20 implanted in an osteotomy 64 in a tibial tuberosity of tibia 52 of a subject, in accordance with some applications of the present invention.

Typically, prior to insertion of the implant, osteotomy 64 (Fig. 12) is created in the tibia. As shown in Fig. 13, the implant is then provided, and is inserted, along an axis of insertion 66, into the osteotomy. By inserting the implant in the osteotomy, tibial tuberosity 58 is rotated about axis of insertion 66, which results in a change in the angular orientation of the tibial tuberosity with respect to the other portion of the tibia. (As noted above, this rotation is facilitated by the distance between the top and bottom of the implant being greater at one side than at the other side.) Patellar tendon 56 transfers this rotational effect to patella 54, such that the patella is indirectly also rotated about axis of insertion 66, by an angle theta. Thus, the angular orientation of patella 54 is changed with respect to femur 50, and in particular, with respect to the trochlea 68 of the femur. As shown in Fig. 12, the insertion of the implant typically indirectly also increases a distance D6 of the patella from the trochlea, by pivoting the tibial tuberosity with respect to a point of contact 70 between the tibial tuberosity and the other portion of the tibia. (The pivoting of the tibial tuberosity increases a distance of the proximal portion of the tibial tuberosity from the other portion of the tibia, thus, via the patellar tendon, causing an increase in distance D6.)

As noted above, bone graft material, which is typically coupled to the implant by being inserted into the pores , is typically also inserted into the osteotomy, in order to help maintain the tibial tuberosity in its new position.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

## Claims

1. An apparatus for placement at an anatomical site, the apparatus comprising:
an orthopedic implant, comprising:
a front end,
a rear end,
a top,
a bottom,
a right side,
a left side, and
a central part connecting the right and left sides,
wherein the top of the implant has a top surface defining a first sloping surface running downwardly from the front end to the rear end of the implant, and the implant having a second sloping surface running downwardly from one side to the opposite side of the implant, and wherein the first and second sloping surfaces are perpendicular to each other;
at least a portion of the implant being shaped to define a porous matrix, the porous matrix being shaped to define a plurality of pores that extend through the porous matrix,the pores being sized to receive inserted bone graft material into the pores; and
wherein at least the central part of the implant has a plurality of said pores therein, and the central part defines at least one fixation passageway that passes through the implant from the top of the implant to the bottom of the implant, the at least one fixation passageway having a diameter larger than an average diameter of the pores and is extending almost perpendicular to said second sloping surface running downwardly from one side to the opposite side of the implant, and is adapted to accept a fixation element.

2. The apparatus according to claim 1, wherein at least one of the right and left sides of the implant is shaped to define at least one further passageway that passes through the side, the at least one further passageway having a diameter larger than an average diameter of the pores.

3. The apparatus according to claim 1, wherein at least 90 percent of a volume of the portion of the implant that includes the pores comprises the porous matrix.

4. The apparatus according to claim 1, wherein at least a portion of the front end, the rear end, the top, the bottom, the right side, or the left side of the implant defines a portion of the porous matrix.

5. The apparatus according to claim 1, wherein the implant is adapted for use with a tool, and the implant further comprises a connection interface that facilitates a connection of the implant to the tool.

6. The apparatus according to claim 5, wherein the connection interface is front-facing.

7. The apparatus according to claim 5, wherein the connection interface is shaped to define a threaded hole.

8. The apparatus according to claim 5, wherein the tool comprises a rod that is fixed to the implant at the connection interface.

9. The apparatus according to claim 8, wherein a connection between the rod and the implant is configured to be breakable at the connection interface due to a size of the connection interface.

10. The apparatus according to claim 1, wherein the distance between the top and the bottom is greater at the front end than at respective corresponding opposite locations on the rear end, on both of the left and the right sides of the implant.

11. The apparatus according to claim 1, further comprising a bone graft material.

12. The apparatus according to claim 1, wherein the implant is made of a metal.

13. The apparatus according to claim 1, wherein the implant is made of a plastic material.

14. The apparatus according to claim 1, wherein the plurality of pores extending through the porous matrix extend through the porous matrix in at least each of: (a) a front-to-rear direction, (b) a left-to-right direction, and (c) a top-to-bottom direction.

15. The apparatus according to claim 1, wherein the diameter of the pores of the porous matrix is about 0.5 mm.
